# EUROPEAN PATENT SPECIFICATION

(11) **EP 1 787 599 B1**
(45) Date of publication and mention of the grant of the patent: **04.08.2010**
(21) Application number: 05447256.8
(22) Date of filing: 18.11.2005
(51) Int. Cl.: A61B 19/08, A61B 17/02

(54) **Cover for wrapping a surgical instrument**
Überzug zum Umhüllen eines chirurgischen Instrumentes
Elément pour envelopper un appareil chirurgical

(43) Date of publication of application: 23.05.2007
(73) Proprietor: NV Hoste Industries, 9800 Deinze (BE)
(72) Inventor: Van den Heede, Thérèse, 9750 Huise (BE); Hoste, Bernard, 9810 Nazareth (BE)
(74) Representative: Vanhalst, Koen

(56) References cited:
- WO-A-96/25875
- US-A- 3 882 855
- US-A- 4 884 558
- US-A- 4 972 825
- US-A- 5 709 646
- US-B1- 6 594 971

## Description

### Technical field

The present invention relates to the field of medical instruments, particularly to surgical instruments, and more particularly to an elastic cover for wrapping said surgical instrument and a method for fabricating such cover.

### Background

Modern surgery requires adequate exposure in the surgical area to provide sufficient visual and physical access to the organs. This is ordinarily accomplished by the use of hand-held or self-retaining retractors comprised of a blade area for engaging the tissue at the point of incision, a retention means, and a shank, which connects the blade to the retention means. The retractor blades come in a multitude of shapes and sizes depending upon the surgical procedure to be performed. There are also a multitude of retention means, which range from self-retaining devices to handles, the numerous shapes of which have been dictated by the preferences of surgical nurses and assistants.

Surgical retractors are generally made from stainless steel because of its strength and its ability to be sterilized. The retractors are therefore extremely hard and become very slippery when they come into contact with blood and other bodily fluids during the surgical procedures. This hardness also often results in tissue damage and bruising due to the pressure that must be exerted during retraction of these tissues. Stainless steel is also thermally highly conductive and quickly absorbs heat from the tissue with which it comes into contact, often resulting in tissue injury. A further disadvantage of standard stainless steel retractors is that their reflective surfaces produce glare under the high level illumination typical during surgical procedures.

Another problem is that retractor handles can also become slippery during use and difficult to hold onto. They are also very uncomfortable because of their hardness.

In an effort to alleviate some of these many disadvantages of stainless steel retractors, nurses often wrap the retractors with gauze, which is then held in place with rubber bands or tape.

Alternatively, US 5,709,646 for instance discloses surgical retractor covers, which may be applied without the use of tape or the like.

Patent document US 6,594,971 discloses an enclosure for receiving an endoscope during a sterilization procedure and sterile storage thereafter. The preamble of claim 1 is based on this document.

Patent document WO 96/25875 discloses a cover for wrapping a laryngoscope comprising an elastic sheath that substantially conforms to the exterior surface of the laryngoscope.

Patent document US 4,972,825 discloses a laryngoscope cover having a body with a hollow interior for receiving a laryngoscope; wherein the body comprises a closed first end, an open second end and a closing structure for closing the second end of the body.

The disclosed covers are suitable for covering either the blade part of an instrument or the handle end of hand-held instruments.

However, a problem associated with the use of this type of wraps is that application of the wraps on the instruments is very time-consuming. A nurse generally has to cut the wraps to a suitable size, stitch the wrap to a suitable configuration and sometimes also sterilise the wrap before applying it on the instrument. Moreover, wrapping of the instruments with the wraps sometimes needs to be done under sterile conditions.

Another problem is that the above-described types of wraps for surgical instruments can cause complications if the wrapping comes apart and spills within the body cavity. Another problem of such wraps is that they generally lack sufficient stretching power to effectively and tightly cover the instruments, which hampers an adequate use of the instruments.

The present invention aims to provide a solution to at least some of the above-mentioned problems.

More in particular, the present invention aims to provide a cover wrap, which is particularly suitable for entirely covering a surgical instrument

It is another object of the present invention to provide a cover wrap which can be adequately and tightly stretched over the entire instrument and which shows sufficient stretching power over the entire surface of the instrument.

### Summary

The present invention teaches a novel disposable, elastic sheath which can be secured over the entire surface of surgical instruments such as surgical retractors to reduce slipping of the instrument in the body cavity, reduce trauma to tissue, and reduce glare. More in particular, the present invention relates to a cover for wrapping a hand-held surgical instrument prior to a sterilization process, comprising: a substantially tubular elastic sheath provided with an aperture for the insertion of said surgical instrument, which sheath is suitably dimensioned for essentially entirely wrapping said surgical instrument, and a cover element which is attached to one end of said tubular sheath and capable of covering said aperture of said tubular sheath, according to claim 1.

In another aspect, the invention relates to the use of a cover according to the invention for wrapping a surgical instrument, according to claims 14 and 15.

The present cover enables to reduce the slipperiness of surgical instruments during use and greatly improves handling of the instruments. In addition, because the present cover is elastic and suitable for entirely wrapping the surgical instruments, it tightly and closely covers the instrument and provides a sufficient stretching power over the entire surface of the instrument. As such the material has sufficient stretchability to be tightly stretched over the different widths and lengths of the instruments without causing folds or crinkles, which could hamper correct use of the surgical instrument. Another advantage of providing a cover that is wrapped over the entire surface of a surgical instrument is that problems with regard to spills of the cover or parts thereof within the body cavity are significantly reduced.

In another aspect, the present invention provide a cover which is fabricated in such a manner that the cover is elastic and smooth and does not show any irregularities, wrinkles or seams when applied over a surgical instrument. More in particular, the invention is related to method for fabricating a cover for wrapping a surgical instrument according to claim 13, comprising the steps of:
a) providing a substantially tubular elastic sheath having open side ends;
b) closing the upper side end of the tubular sheath obtained in step a) by stitching;
c) turning the tubular sheath obtained in step b) inside out;
d) making an lateral aperture at the lower side end of the tubular sheath obtained in step c);
e) closing the lower side end of the tubular sheath obtained in step d) by stitching;
f) providing a cover element, of which the top is closed and of which the basis is provided with an open end, and
g) attaching the top of said cover element to the lower end of the tubular sheath which is provided with a lateral aperture in accordance with step d).

Covers with different dimensions may advantageously be fabricated for different types and dimensions of surgical instruments. The present invention thus provides covers, which are standardised according to the type of instrument. In addition, the cover can be very rapidly and easily fabricated. The present covers can be wrapped onto surgical instruments in a minimum of time without using any stitching means. Also, wrapping of a surgical instrument with the present cover need not be done under sterile conditions, since the wrapped instrument can be easily sterilised prior to use. Another advantage of the present invention is that a cover is made of an elastic material and that it remains elastic, even after a sterilisation process.

These and other advantages of the invention will become clearer upon study of the following description when read in conjunction with the appended drawings.

### Description of the figures

**FIG. 1** represents an embodiment of a hand-held surgical instrument.
**FIG. 2** illustrates a preferred embodiment of a cover for wrapping a surgical instrument according to the invention.
**FIG. 3** illustrates the method for wrapping a hand-held surgical instrument with a cover according to the present invention. **FIG. 3a** represents introduction of the surgical instrument in the cover through the lateral aperture present in the tubular sheath part of the cover. **FIG. 3a** illustrates how the cover element of a cover according to the invention is applied on the handle of the surgical instrument.
**FIG. 4** represents a surgical instrument, which has been entirely wrapped with a cover according to the present invention.

### Detailed description of the invention

The present invention relates a cover that may be used to wrap any type of surgical instruments. In a preferred embodiment, the present covers are in particular suitable for wrapping surgical instruments, which are used for retracting and/or lifting organs during surgery, especially during abdominal surgery.

In a first embodiment, the present invention relates to a cover for wrapping a hand-held surgical instrument prior to a sterilization process. The cover comprises a substantially tubular elastic sheath provided with an aperture for the insertion of the surgical instrument. The sheath is suitably dimensioned for essentially entirely wrapping the surgical instrument. The cover further comprises a cover element which is attached to one end of said tubular sheath and capable of covering the aperture of the tubular sheath. According to a preferred embodiment, the tubular sheath of said cover is provided with closed side ends and a lateral aperture. In another preferred embodiment, the said cover element of said cover is provided with a closed top and an open basis. The invention relates in particular to a cover comprising a substantially tubular elastic sheath having the top of said cover element attached to one end thereof. Most preferably, the lateral aperture of the tubular sheath is provided at the side of attachment of the cover element to the tubular sheath.

In another embodiment, the tubular sheath and the cover element of a cover according to the invention comprise a fabric. In a preferred embodiment, this fabric comprises at least two layers and/or at least two directions of individual elements, wherein the individual elements in at least one, and preferably in both layers and/or directions of the fabric are made of an elastic material. These fabrics might contain a warp and/or a weft.

According to the invention an "individual element" implies a warp or a weft element. A "warp element" is to be understood as one or more individual elements such as e.g. yarns, filaments, bundles of fibers, wires or cords, which follow the same path through the fabric in the warp direction. A "weft element" is to be understood as one or more individual elements such as e.g. yarns, filaments, bundles of fibers, wires or cords, which follow the same path through the fabric in the weft direction. The "warp" comprises different warp elements, laying in a same direction, the so-called "warp direction". The "weft" comprises different weft elements, laying in a same direction, the so-called "weft direction". Each warp and weft element follows a certain path through the fabric, being respectively a warp path or a weft path. Preferably, but not necessarily, all individual elements of a warp element cross the weft elements of the fabric in an identical way. Also, preferably, but not necessarily, all individual elements of a weft element cross the warp elements of the fabric in an identical way. The term "directions" may herein refer to a weft direction, a warp direction and/or a bias direction. The term "bias direction" implies a direction diagonally across a piece of fabric at preferably 45 degrees to the warp. In the present invention the degrees to the warp may differ from 45, in a range of from 1 to 89 degrees, for example 10, 20, 30, 40, 50, 60, 70 or 80 degrees.

According to another embodiment, the present cover or fabric comprises at least two layers and/or at least two directions of individual elements, which might be laid upon each other by weaving, stitching or knitting techniques. The individual elements in the fabric can be interwoven as long as this weaving, stitching, suing, or whatever textile construction allows sufficient elasticity to the individual yarns. In another embodiment, said fabric may be a woven fabric. In an embodiment, said fabric may be a knitted fabric. "Knitting means" implies a method for forming a fabric or textile surface produced by interlacing stitches (loops). The term knitting may comprise flat knitting or round knitting or a combination thereof. It shall be clear that the cover element and the tubular sheath of the cover may be made by a same or by a different procedure. For instance, the cover element may be a knitted fabric while the tubular sheath may be a woven fabric, or *vice versa*. In another embodiment, the cover element and the tubular sheath may both be a woven or a knitted fabric.

According to the present invention, the individual elements in the warp and/or weft layers and/or directions are made of an elastic material. The term "elastic material" refers to a material that may have a total length, which in stretched condition is much larger than its length in non-stretched condition, without causing any rupture or crack in the material. More in particular, a cover according to the present invention is characterised by a total length, which in stretched condition is up to 30%, preferably up to 80%, preferably up to 100 %, preferably up to 200%, preferably up to 400%, preferably up to 600 % or preferably up to 750% of the length of the cover in a non-stretched condition. Due to its elasticity, the present cover may advantageously be smoothly and tightly applied on a surgical instrument, thereby providing optimal stretching power and without causing undesired wrinkles in the material.

In a preferred embodiment, the cover according to the invention is made of an elastic material, preferably selected from the group comprising natural fibres such as cotton, wool, bamboo, linen, silk, hemp, jute, modal, etc... and/or synthetic fibres such as elasthane, viscose, polyamide, or other elastic polymer fibres, etc... Advantageously, the cover according to the present invention is made of a material that remains elastic after a sterilisation process.

The terms "fibre" or "yarn" are used herein as synonyms. In a particularly preferred embodiment, the invention provides a fabric comprising a warp and a weft. The weft and the warp may comprise simple yarns and/or twined yarns. Yarns that are twisted are called twined yarns. The term "twined" should be understood in its broadest context and comprises for instance also simple twisting of the yarns. The yarn may be twined in the S-or Z-direction. The fibers may include natural fibers, synthetic fibers or combinations thereof. For instance, the natural fibers or yarns may be completely or partly mixed with other fibers or yarns, for instance with synthetic fibers. Mixing of natural fibers with synthetic fibers may be done for simple as well as for wined yarns.

In a preferred embodiment the material may be made of 25 to 100 %, and preferably of 50 to 100% natural fibers. In another embodiment, the material may be made of 25 to 100%, and preferably of 50 to 100 % synthetic fibers. Suitable examples of natural fibers include but are not limited to cotton, wool, bamboo, linen, silk, hemp, jute, modal. Suitable examples of synthetic fibers include but are not limited to viscose, polyamide, elasthane or other elastic polymer fibers. In another embodiment, the material may be made of a combination of natural and synthetic fibers. In an example, the ratio of the amount of natural to synthetic fibers in such material is comprised between 1:4 and 4:1.

In a preferred embodiment, the present cover may consist of a fabric comprising a warp and a weft. In an embodiment, a fabric is provided wherein the warp consists of 25 to 100 %, and preferably of 50 to 100% natural elastic fibers and/or of 25 to 100 %, and preferably of 50 to 100% synthetic elastic fibers. In another embodiment, the present cover consists of a fabric wherein the weft consists of 25 to 100 %, and preferably of 50 to 100% natural elastic fibers and/or of 25 to 100 %, and preferably of 50 to 100% synthetic elastic fibers. In yet another embodiment a fabric is provided wherein the warp and the weft consists of 25 to 100 %, and preferably of 50 to 100% natural elastic fibers and/or of 25 to 100 %, and preferably of 50 to 100% synthetic elastic fibers.

According to a further embodiment, the tubular sheath and the cover element of a cover according to the present invention are made of a same material. Alternatively, the tubular sheath and the cover element of a cover according to the present invention are made of a different material. The cover element may be provided in an elastic material which improves its function as a grip or handle, and can for instance be made of elastic synthetic material such as polyamide, elasthane, polyurethane foam or the like.

In addition, the cover element and the tubular sheath of the present cover may have a different degree of elasticity. In yet another further embodiment, the cover of the present invention may be provided in different colours. It shall further be clear that the tubular sheath and the cover of the present invention may be provided in a same colour or in different colours.

The cover according to the present invention is further characterised by the following technical characteristics.

A cover according to the present invention may have various dimensions and weights, adapted to wrap different types of surgical instruments. Preferably a cover according to the invention has a weight comprised between 3 gram and 30 gram and for instance between 3 gram and 10 gram, or between 10 gram and 20 gram.

In a preferred embodiment, a cover according to the invention is provided wherein said tubular sheath has a length comprised between 10 and 500 mm, and preferably between 100 and 300 mm. In yet another preferred embodiment a cover according to the invention has a cover element having a length comprised between 10 and 100 mm, and preferably between 10 and 50 mm. Preferably, the ratio of the length of the cover element to the length of the tubular sheath is comprised between 1:2 and 1:10, and preferably is comprised between 1:2 and 1:5.

In another preferred embodiment, the elastic material of the tubular sheath has a thickness comprised between 0.5 mm and 10 mm, and for instance between 0.6 and 7 mm, or between 2 and 5 mm. In addition, the material of the cover element preferably has a thickness comprised between 0.5 mm and 10 mm, and for instance between 0.6 and 7 mm, or between 2 and 5 mm.

In yet another embodiment, the present cover according to the present invention is provided with a tubular sheath having a lateral aperture at one of its side ends. Preferably, said lateral aperture in said tubular sheath has a length comprised between 5 and 100 mm, and preferably between 10 and 50 mm. The size of the aperture is chosen so as to enable proper and easy engagement of a surgical instrument into the tubular sheath of the cover.

A cover according to the present invention is sterilisable up to 140 °C. For instance, it was shown that the present instrument, in wrapped condition, could be sterilised for 40 minutes at 135°C without any loss of elasticity of the wrapping cover. Preferably, a cover according to the present invention is disposed after a single use.

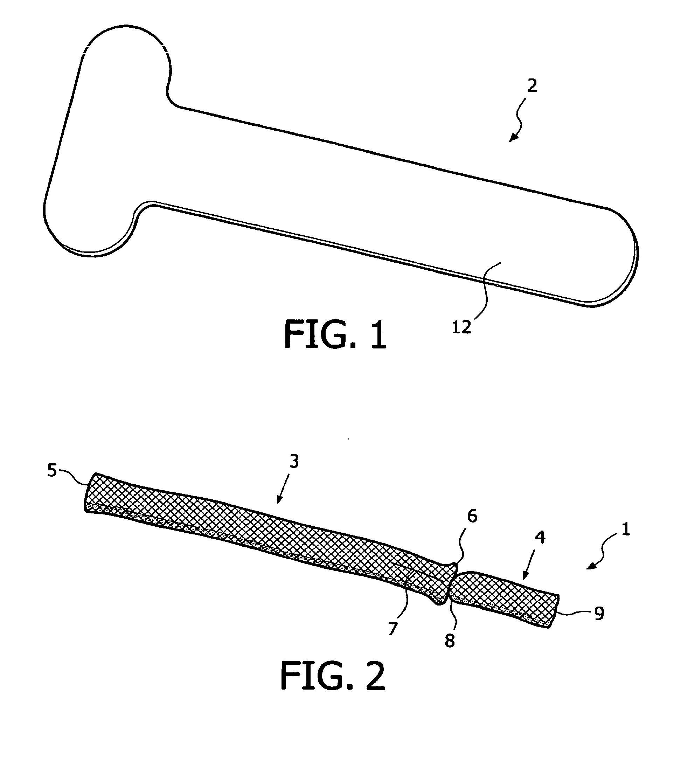

Referring now to the drawings, a preferred embodiment of a cover 1 according to the invention is represented in **FIG. 2****.** The represented cover is for instance particularly suitable for wrapping a surgical instrument 2 as illustrated on **FIG. 1****.** The instrument may be a typical, surgical hand-held instrument 2. The cover 2 comprises a substantially tubular sheath 3 suitably dimensioned for essentially entirely wrapping a surgical instrument 2, which is further provided with a cover element 4 attached to one end of said tubular sheath, preferably to the lower side end of the tubular sheath. The tubular sheath has an upper 5 and a lower 6 side end which are closed by stitching. The cover element 4 is provided with a closed top 8 and an open basis 9. The top 8 of said cover element is attached to the lower side end 6 of the tubular sheath. The tubular sheath further comprises a lateral aperture 7 at the lower side 6 thereof.

The present cover for wrapping a hand-held surgical instrument may be fabricated by the steps of:
a) providing a substantially tubular elastic sheath 3 having open side ends 5,6;
b) closing the upper side end 5 of the tubular sheath obtained in step a) by stitching;
c) turning the tubular sheath obtained in step b) inside out;
d) making an lateral aperture 7 at the lower side end 6 of the tubular sheath 3 obtained in step c);
e) closing the lower side end 6 of the tubular sheath obtained in step d) by stitching;
f) providing a cover element 4, of which the top 8 is closed and of which the basis 9 is provided with an open end, and
g) attaching the top 8 of said cover element to the lower end 6 of the tubular sheath which is provided with a lateral aperture 7 in accordance with step d).

Alternatively, step d) precedes step c) of the above-mentioned process.

In a first step, a tubular sheath having open lower and upper side ends is provided. Such tubular sheath can for instance be a knitted fabric, for instance obtained by round or flat knitting techniques. The tubular sheath will be stitched at its side ends in order to have closed side ends. In first instance, the upper side end of the tubular sheath is closed by stitching. The sheath will be subsequently turned inside out, such that the stitching seams at the upper side end of the sheath are located at the inside of the tubular sheath and such that the outside of the tubular sheath is smooth. During the process of turning the sheath inside out, a lateral incision will be made in the sheath material, by means of a sharp knife, more in particular at the lower side end of the tubular sheath, in order to obtain a relatively small and precise lateral aperture in the tubular sheath. Subsequently, the lower side end of the tubular sheath will be closed by stitching. To hide the aperture and the stitching seams on the lower side end of the sheath, a cover element is provided, of which the top is closed and of which the basis is provided with an open end. The top of this cover element is attached by means of stitching to the lower side end of the tubular sheath. The cover element can be engaged over at least a part of the handle of the surgical instrument by turning the cover element inside out, such that the cover element covers loose threads of the aperture and the seam of the lower sheath side.

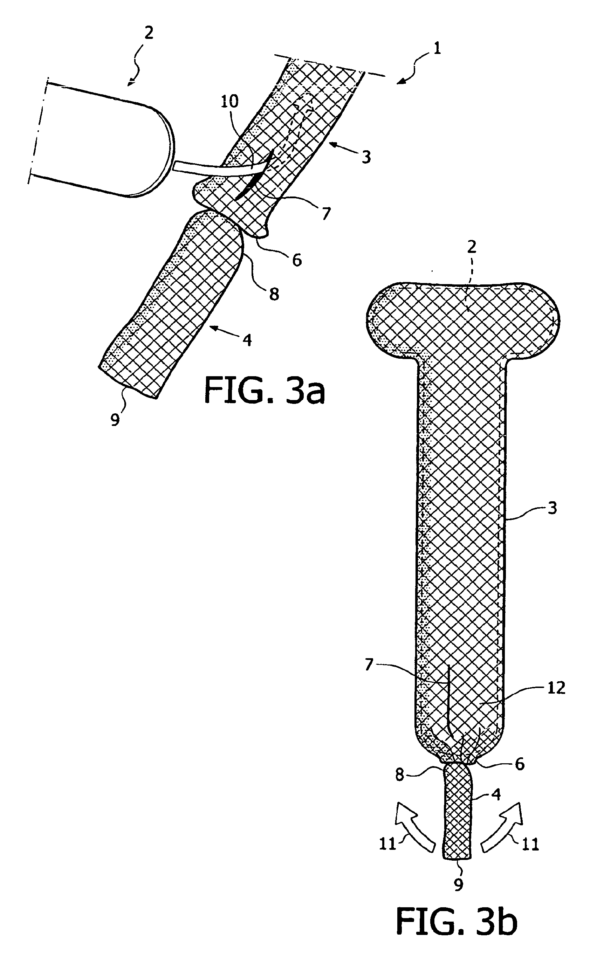

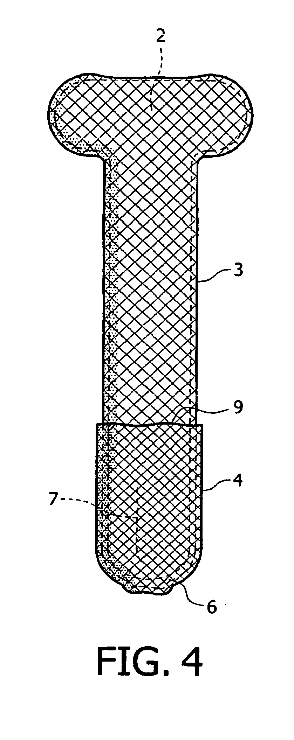

**FIG. 3** illustrates the wrapping of a cover 1 according to the present invention on a hand-held surgical instrument. Wrapping involves engaging (indicated by arrow 10) the surgical instrument 2 through the aperture 7 in the tubular sheath 3 of said cover 1 as illustrated in **FIG. 3a****,** and entirely covering the surgical instrument with the tubular sheath. In a next step, the cover element 4 of said cover 1 is turned inside out as indicated by arrow 11 in **FIG. 3b** while engaging said cover element 4 over the lower part 12 of the surgical instrument in order to cover the aperture 7 of the tubular sheath 3. As illustrated on **FIG. 3b** and **FIG. 4** the cover element 2, which is engaged over the lower part of the surgical instrument, completely covers the aperture 7 and the seam 6 of the lower sheath side, so as to provide a wrapped instrument having a smooth and tight surface.

Those skilled in the art will recognize that many modifications and variations of the present invention are possible in light of the above teachings. It is therefore to be understood that, within the scope of the appended claims, the invention may be practiced otherwise than as specifically described.

## Claims

1. Cover (1) for wrapping a hand-held surgical instrument (2) prior to a sterilization process, comprising:
- a substantially tubular sheath (3) provided with closed side ends (5, 6) and a lateral aperture (7) for the insertion of said surgical instrument (2), which sheath (3) is suitably dimensioned for essentially entirely wrapping said surgical instrument, and
- a cover element (4) which is attached to one end of said tubular sheath (3) and capable of covering said aperture (7) of said tubular sheath (3)
wherein said lateral aperture (7) is provided at the side of attachment of the cover element (4) to the tubular sheath (3),
**characterised in that** the tubular sheath (3) is elastic and **in that** said cover element (4) is provided with a closed top (8) attached to one end (6) of said tubular sheath and an open basis (9), wherein the aperture (7) is covered by turning the cover element (4) inside out.

2. Cover according to claim 1, wherein said tubular sheath and said cover element comprise a fabric, preferably a knitted or woven fabric.

3. Cover according to claims 1 or 2, wherein said fabric comprises at least two layers and/or at least two directions of individual elements and wherein the individual elements in at least one, and preferably in both layers and/or directions of the fabric are made of an elastic material.

4. Cover according to any of claims 1 to 3, wherein said elastic material is selected from the group comprising natural fibers and/or synthetic fibers.

5. Cover according to any of claims 1 to 4, wherein the elastic material comprises 25 to 100 % natural fibers and /or 25 to 100 % synthetic fibers.

6. Cover according to any of claims 1 to 5, wherein the cover has a total length, which in stretched condition is up to 30%, preferably up to 100 % and even more preferred up to 200% of the length in of the cover in non-stretched condition.

7. Cover according to any of claims 1 to 6, wherein said tubular sheath has a length comprised between 10 and 500 mm.

8. Cover according to any of claims 1 to 7, wherein said cover element has a length comprised between 10 and 100 mm.

9. Cover according to any of claims 1 to 8, wherein said lateral aperture in said tubular sheath has a length comprised between 5 and 100 mm.

10. Cover according to any of claims 1 to 9, wherein the ratio of the length of the cover element to the length of the tubular sheath is comprised between 1:2 and 1:10.

11. Cover according to any of claims 1 to 10, wherein said cover has a weight comprised between 3 and 30 gram.

12. Cover according to any of claims 1 to 11, wherein said cover is sterilisable up to 140 °C.

13. Method for fabricating a cover according to any of claims 1-12 for wrapping a surgical instrument, comprising the steps of:
a) providing a substantially tubular elastic sheath (3) having open side ends (5,6);
b) closing the upper side end (5) of the tubular sheath obtained in step a) by stitching;
c) turning the tubular sheath obtained in step b) inside out;
d) making an lateral aperture (7) at the lower side end (6) of the tubular sheath (3) obtained in step c);
e) closing the lower side end (6) of the tubular sheath obtained in step d) by stitching;
f) providing a cover element (4), of which the top (8) is closed and of which the basis (9) is provided with an open end, and
g) attaching the top (8) of said cover element to the lower end (6) of the tubular sheath which is provided with a lateral aperture (7) in accordance with step d).

14. Use of a cover (1) according to any of claims 1 to 12 for wrapping a surgical instrument (2).

15. Method for wrapping a surgical instrument (2) with a cover (1) according to any of claims 1 to 12 comprising the steps of:
a) engaging the surgical instrument (2) through the aperture (7) in the tubular sheath (3) of said cover (1) and entirely covering the surgical instrument with the tubular sheath, and
b) turning the cover element (4) of said cover inside out while engaging said cover element over the lower side end (12) of the surgical instrument in order to cover the aperture (7) of the tubular sheath (3).

## Patentansprüche

1. Hülle (1) zum Einpacken eines chirurgischen Handgeräts (2) vor einem Sterilisationsverfahren, umfassend:
- eine im Wesentlichen schlauchförmige Umhüllung (3), die mit geschlossenen Seitenenden (5, 6) und einer seitlichen Öffnung (7) für das Einführen des chirurgischen Instruments (2) ausgestattet ist, wobei die Umhüllung (3) angemessen dimensioniert ist, um im Wesentlichen das chirurgische Instrument vollständig einzuhüllen, und
- ein Hüllelement (4), das an einem Ende von der schlauchförmigen Umhüllung (3) befestigt ist und mit dem es möglich ist, die Öffnung (7) in der schlauchförmigen Umhüllung (3) zu bedecken,
wobei die seitliche Öffnung (7) an der Seite der Befestigung von dem Hüllelement (4) an die schlauchförmige Umhüllung (3) bereitgestellt ist, **dadurch gekennzeichnet, dass** die schlauchförmige Umhüllung (3) elastisch ist und dadurch, dass das Hüllelement (4) mit einer geschlossenen Kopfseite (8), die an einem Ende (6) von der schlauchförmigen Umhüllung befestigt ist, und einer offenen Unterseite (9) versehen ist, wobei die Öffnung (7) durch Umkrempeln des Hüllelements (4) bedeckt wird.

2. Hülle nach Anspruch 1, wobei die schlauchförmige Umhüllung und das Hüllelement ein Gewebe, vorzugsweise einen gewirkten oder gewebten Stoff umfassen.

3. Hülle nach Anspruch 1 oder 2, wobei das Gewebe mindestens zwei Lagen und/oder mindestens zwei Richtungen der individuellen Gewebeelemente umfasst und wobei die individuellen Gewebeelemente in mindestens einer Lage und vorzugsweise in beiden Lagen und/oder Richtungen aus einem elastischen Material hergestellt sind.

4. Hülle nach einem der Ansprüche 1 bis 3, wobei das elastische Material ausgewählt ist aus der Gruppe, die natürliche Fasern und/oder synthetische Fasern umfasst.

5. Hülle nach einem der Ansprüche 1 bis 4, wobei das elastische Material 25 bis 100 % natürliche Fasern und/oder 25 bis 100 % synthetische Fasern umfasst.

6. Hülle nach einem der Ansprüche 1 bis 5, wobei die Hülle eine Gesamtlänge aufweist, die im gedehnten Zustand bis zu 30 %, vorzugsweise bis zu 100 % und noch bevorzugter bis zu 200 % der Länge der Hülle im ungedehnten Zustand aufweist.

7. Hülle nach einem der Ansprüche 1 bis 6, wobei die schlauchförmige Umhüllung eine Länge umfasst, die zwischen 10 und 500 mm beträgt.

8. Hülle nach einem der Ansprüche 1 bis 7, wobei das Hüllelement eine Länge umfasst, die zwischen 10 und 100 mm beträgt.

9. Hülle nach einem der Ansprüche 1 bis 8, wobei die seitliche Öffnung in der schlauchförmigen Umhüllung eine Länge umfasst, die zwischen 5 und 100 mm beträgt.

10. Hülle nach einem der Ansprüche 1 bis 9, wobei das Verhältnis der Länge von dem Hüllelement zu der Länge von der schlauchförmigen Umhüllung zwischen 1:2 und 1:10 beträgt.

11. Hülle nach einem der Ansprüche 1 bis 10, wobei die Hülle ein Gewicht aufweist, das zwischen 3 und 30 Gramm liegt.

12. Hülle nach einem der Ansprüche 1 bis 11, wobei die Hülle bei bis zu 140 °C sterilisierbar ist.

13. Verfahren zur Anfertigung einer Hülle nach einem der Ansprüche 1 bis 12 zum Einhüllen eines chirurgischen Instruments, das die Schritte umfasst:
a) Bereistellen einer im Wesentlichen schlauchförmigen, elastischen Umhüllung (3), die offene Seitenenden (5, 6) aufweist;
b) Verschließen des oberen Seitenendes (5) von der schlauchförmigen Umhüllung, die in Schritt a) erhalten wurde, durch Nähen;
c) Umkrempeln der schlauchförmigen Umhüllung, die in Schritt b) erhalten wurde;
d) Herstellen einer seitlichen Öffnung (7) an dem unteren Seitenende (6) von der schlauchförmigen Umhüllung (3), die in Schritt c) erhalten wurde;
e) Verschließen des unteren Seitenendes (6) von der schlauchförmigen Umhüllung, die in Schritt d) erhalten wurde, durch Nähen;
f) Bereitstellen eines Hüllelements (4), von dem die Kopfseite (8) geschlossen ist und von dem die Unterseite (9) mit einem offenen Ende versehen ist; und
g) Befestigen der Kopfseite (8) von dem Hüllelement an dem unteren Ende (6) von der schlauchförmigen Umhüllung, die gemäß Schritt d) mit einer seitlichen Öffnung (7) versehen ist.

14. Verwendung einer Hülle (1) nach einem der Ansprüche 1 bis 12 zum Einpacken eines chirurgischen Instruments (2).

15. Verfahren zum Einpacken eines chirurgischen Instruments (2) in einer Hülle (1) nach einem der Ansprüche 1 bis 12, das die Schritte umfasst:
a) Einführen des chirurgischen Instruments (2) durch die Öffnung (7) in der schlauchförmigen Umhüllung (3) von der Hülle (1) und vollständiges Bedecken des chirurgischen Instruments mit der schlauchförmigen Umhüllung, und
b) Umkrempeln des Hüllelements (4) von der Hülle während das Hüllelement über das untere Ende (12) von dem chirurgischen Instrument geführt wird, um die Öffnung (7) von der schlauchförmigen Umhüllung (3) zu bedecken.

## Revendications

1. Couverture (1) destinée à envelopper un instrument chirurgical manuel (2) avant un processus de stérilisation, qui comprend :
- une gaine sensiblement tubulaire (3) dotée d'extrémités latérales fermées (5, 6) et d'une ouverture latérale (7) pour l'insertion dudit instrument chirurgical (2), laquelle gaine (3) a des dimensions adaptées pour envelopper sensiblement la totalité dudit instrument chirurgical, et
- un élément de couverture (4) qui est fixé à une extrémité de ladite gaine tubulaire (3) et capable de couvrir ladite ouverture (7) de ladite gaine tubulaire (3)
ladite ouverture latérale (7) étant prévue sur le côté de fixation de l'élément de couverture (4) à la gaine tubulaire (3)
**caractérisée en ce que** la gaine tubulaire (3) est élastique et **en ce que** ledit élément de couverture (4) est doté d'un haut fermé (8) fixé à une extrémité (6) de ladite gaine tubulaire et d'une base ouverte (9), l'ouverture (7) étant couverte en retournant l'élément de couverture (4).

2. Couverture selon la revendication 1, dans laquelle ladite gaine tubulaire et ledit élément de couverture comprennent un tissu, de préférence un tissu tricoté ou tissé.

3. Couverture selon les revendications 1 ou 2, dans laquelle ledit tissu comprend au moins deux couches et/ou au moins deux directions d'éléments individuels et dans laquelle les éléments individuels dans au moins un, et de préférence dans les deux couches et/ou directions du tissu sont formés par un matériau élastique.

4. Couverture selon l'une quelconque des revendications 1 à 3, dans laquelle ledit matériau élastique est choisi dans le groupe comprenant des fibres naturelles et/ou des fibres synthétiques.

5. Couverture selon l'une quelconque des revendications 1 à 4, dans laquelle le matériau élastique comprend de 25 à 100 % de fibres naturelles et/ou de 25 à 100 % de fibres synthétiques.

6. Couverture selon l'une quelconque des revendications 1 à 5, la couverture ayant une longueur totale qui, dans une condition étirée, peut atteindre 30 %, de préférence 100 % et idéalement 200 % de la longueur de la couverture dans une condition non étirée.

7. Couverture selon l'une quelconque des revendications 1 à 6, dans laquelle ladite gaine tubulaire a une longueur comprise entre 10 et 500 mm.

8. Couverture selon l'une quelconque des revendications 1 à 7, dans laquelle ledit élément de couverture a une longueur comprise entre 10 et 100 mm.

9. Couverture selon l'une quelconque des revendications 1 à 8, dans laquelle ladite ouverture latérale dans ladite gaine tubulaire a une longueur comprise entre 5 et 100 mm.

10. Couverture selon l'une quelconque des revendications 1 à 9, dans laquelle le rapport de la longueur de l'élément de couverture sur la longueur de la gaine tubulaire est compris entre 1/2 et 1/10.

11. Couverture selon l'une quelconque des revendications 1 à 10, ladite couverture ayant un poids compris entre 3 et 30 grammes.

12. Couverture selon l'une quelconque des revendications 1 à 11, ladite couverture pouvant être stérilisée jusqu'à 140°C.

13. Procédé de fabrication d'une couverture selon l'une quelconque des revendications 1 à 12, pour envelopper un instrument chirurgical, qui comprend les étapes consistant à :
a) fournir une gaine élastique sensiblement tubulaire (3) ayant des extrémités latérales ouvertes (5, 6) ;
b) fermer l'extrémité latérale supérieure (5) de la gaine tubulaire obtenue dans l'étape a) par piquage ;
c) retourner la gaine tubulaire obtenue dans l'étape b) ;
d) former une ouverture latérale (7) au niveau de l'extrémité latérale inférieure (6) de la gaine tubulaire (3) obtenue dans l'étape c) ;
e) fermer l'extrémité latérale inférieure (6) de la gaine tubulaire obtenue dans l'étape d) par piquage ;
f) fournir un élément de couverture (4), dont le haut (8) est fermé et dont la base (9) est dotée d'une extrémité ouverte, et
g) fixer le haut (8) dudit élément de couverture à l'extrémité inférieure (6) de la gaine tubulaire qui est dotée d'une ouverture latérale (7) selon l'étape d).

14. Utilisation d'une couverture (1) selon l'une quelconque des revendications 1 à 12, pour envelopper un instrument chirurgical (2).

15. Procédé d'enveloppement d'un instrument chirurgical (2) avec une couverture (1) selon l'une quelconque des revendications 1 à 12, qui comprend les étapes consistant à :
a) engager l'instrument chirurgical (2) dans l'ouverture (7) dans la gaine tubulaire (3) de ladite couverture (1) et couvrir totalement l'instrument chirurgical avec la gaine tubulaire, et
b) retourner l'élément de couverture (4) de ladite couverture tout en engageant ledit élément de couverture sur l'extrémité latérale inférieure (12) de l'instrument chirurgical de manière à couvrir l'ouverture (7) de la gaine tubulaire (3).
